# EUROPEAN PATENT APPLICATION

(11) **EP 2 103 312 A1**
(43) Date of publication of application: **23.09.2009**
(21) Application number: 07849809.4
(22) Date of filing: 10.12.2007
(51) Int. Cl.: A61K 45/00, A01N 59/00, A61K 33/00, A61P 1/02, A61P 31/04

(54) **PREPARATION FOR STERILIZATION OR DISINFECTION OF TISSUE**

(30) Priority: 12.12.2006 JP 2006334206
(71) Applicant: National University Corporation Tokyo Medical and Dental University, Bunkyo-ku Tokyo 113-8510 (JP); Reo Laboratory Co., Ltd., Higashimatsushima-shi Miyagi 981-0502 (JP)
(72) Inventor: MANO, Yoshihiro, Setagaya-ku Tokyo 157-0061 (JP); ARAKAWA, Shinichi, Setagaya-ku Tokyo 157-0066 (JP); CHIBA, Kaneo, Higashimatsushima-shi Miyagi 981-0502 (JP)
(74) Representative: Duncan, Garreth Andrew
(86) International application number: PCT/JP2007/001376
(87) International publication number: WO 2008/072371

(57) **Abstract**

The present invention relates to a preparation for sterilizing or disinfecting a tissue which has an excellent tissue sterilizing or disinfecting ability and is suitable for therapeutic or prophylactic treatment of various diseases caused by a microorganism such as a bacterium or a virus and a method for sterilizing or disinfecting a tissue. The present invention relates to a preparation for sterilizing or disinfecting a tissue and an agent for therapeutic or prophylactic treatment of a periodontal disease, **characterized by** containing a gas in a nanobubble state. Furthermore, the present invention relates to the above-mentioned preparation for sterilizing or disinfecting a tissue, **characterized in that** the above-mentioned gas in a nanobubble state is ozone. Furthermore, the present invention relates to a liquid preparation for sterilizing or disinfecting a tissue, **characterized by** comprising ozone-nanobubble water.

## Description

### Technical Field

The present invention relates to a preparation for sterilizing or disinfecting a tissue which is suitable for therapeutic or prophylactic treatment of various diseases caused by a microorganism such as bacteria or viruses.

### Background Art

Infection of a tissue with a pathogenic or harmful microorganism such as a bacterium or a virus causes various diseases. It is considered that a drug which can effectively sterilize or disinfect these tissues infected with microorganisms would be very suitable for therapeutic or prophylactic treatment of these various diseases caused by infection with microorganisms.

For example, periodontal disease is a generic name for diseases which occur in periodontal tissues. It is said that various periodontopathic bacteria which exist in plaques: biofilm (for example, *Porphyromonas gingivalis, Aggregatibacter actinomycetecomitans, Tannerella forsythia*, and the like) are pathogens. Periodontal diseases are classified into gingivitis, which is characterized by hemorrhage and swelling due to gingival inflammation, and periodontitis, which is characterized by destruction of the alveolar bone supporting a tooth.

Since periodontal diseases are caused by the above-mentioned periodontopathic bacteria in plaques, the importance of plaque control by daily brushing and the like (prevention of formation of plaques or removal thereof) has been advocated for therapeutic or prophylactic treatment of periodontal diseases. In actual situations, however, many patients suffer from periodontal diseases because of failure of proper plaque control.

Meanwhile, in recent years, water containing a large quantity of oxygen in a nanobubble state has drawn attention because of various bioactive actions thereof on living organisms, such as improving adaptability of fish and shellfish to environmental changes and rapidly recovering debilitated individuals. Nanobubbles are gas bubbles having a nanosize diameter (1 nm or greater and smaller than 1000 nm, for example, smaller than 100 nm), which are even smaller than microbubbles, bubbles having a micro-size diameter (1 µm or greater and smaller than 1000 µm). Nanobubbles are usually generated during the process where microbubbles having a diameter of approx. 50 µm or smaller are reduced in size and have been thought to have a short life generally since they are self-pressurized by the action of surface tension and rapidly dissolved completely. However, it has been reported that even nanosize bubbles can exist for a certain length of time when they are covered with surfactant shells or subject to electrostatic repulsion due to surface charging. A method for producing an aqueous solution which stably contains oxygen or ozone in a nanobubble state for a long period has also been established. In particular, nanobubbles stabilized by the charging effect retain characteristics of gas bubbles, and potentials for application in many fields, such as direct action on an organism at the cellular level, are expected (for example, refer to Patent Document 1). Patent Document 1: Japanese Patent Laid-Open No. 2005-245817

### Disclosure of the Invention

### Technical Problem

An object of the present invention is to provide a preparation for sterilizing or disinfecting a tissue which solves the above-described various problems in the prior art, has an excellent tissue sterilizing or disinfecting ability, and is suitable for therapeutic or prophylactic treatment of various diseases caused by a microorganism such as bacteria or viruses.

### Solution to Problem

To achieve the foregoing object, the inventors of the present invention conducted researches eagerly. As a result, they found that nanobubble water containing a gas in a nanobubble state exhibited excellent effects of improving clinical symptoms of periodontal diseases (gingivitis, periodontitis) and could be suitably used as an excellent preparation for sterilizing or disinfecting a tissue in the field of health care, medical experiments, and the like. This is a novel finding by the inventors of the present invention which has not been known so far.

The present invention was accomplished based on the above-mentioned findings by the present inventors, and the means for solving the above-mentioned problems are as follows, that is to say:
<1> a preparation for sterilizing or disinfecting a tissue, characterized by comprising a gas in a nanobubble state;
<2> the preparation for sterilizing or disinfecting a tissue according to the above <1>, **characterized in that** the gas in a nanobubble state is ozone;
<3> a liquid preparation for sterilizing or disinfecting a tissue, characterized by comprising ozone-nanobubble water.
<4> an agent for therapeutic or prophylactic treatment of a periodontal disease, characterized by comprising a gas in a nanobubble state;
<5> the agent for therapeutic or prophylactic treatment of a periodontal disease according to the above <4>, **characterized in that** the gas in a nanobubble state is ozone;
<6> an agent for therapeutic or prophylactic treatment of a periodontal disease, characterized by comprising ozone-nanobubble water;
<7> a method for sterilizing or disinfecting a tissue, characterized by applying an effective amount of a gas in a nanobubble state;
<8> the method for sterilizing or disinfecting a tissue according to the above <7>, **characterized in that** the gas in a nanobubble state is ozone.
<9> a method for sterilizing or disinfecting a tissue, characterized by applying an effective amount of ozone-nanobubble water;
<10> a method for therapeutic or prophylactic treatment of a periodontal disease, characterized by applying an effective amount of a gas in a nanobubble state;
<11> the method for therapeutic or prophylactic treatment of a periodontal disease according to the above <10>,
   **characterized in that** the gas in a nanobubble state is ozone.
<12> a method for therapeutic or prophylactic treatment of a periodontal disease, characterized by applying an effective amount of ozone-nanobubble water;
<13> use of a gas in a nanobubble state for producing a preparation for sterilizing or disinfecting a tissue;
<14> the use according to claim 13, wherein the gas in a nanobubble state is ozone; and
<15> use of a gas in a nanobubble state for producing an agent for therapeutic or prophylactic treatment of a periodontal disease.

### Advantageous Effects of Invention

According to the present invention, there can be provided a preparation for sterilizing or disinfecting a tissue and a method for sterilizing or disinfecting a tissue, which can solve various problems in the prior art, has an excellent tissue sterilizing or disinfecting ability, and is suitable for therapeutic or prophylactic treatment of various diseases caused by a microorganism such as a bacterium or virus. Particularly in the application to periodontal diseases, the preparation for sterilizing or disinfecting a tissue of the present invention has been found to have not only an effect of killing periodontopathic bacteria but also an effect of regenerating periodontal tissues. Therefore, the present invention is very suitable for prophylactic and therapeutic treatment of these diseases.

Furthermore, when carp with infectious dermatitis and the like were kept in oxygen-nanobubble water, the infectious dermatitis was completely cured. Therefore, it was found that not only ozone-nanobubble water, but also oxygen-nanobubble water had effects of disinfecting, repairing, and regenerating tissues. Furthermore, these facts support a finding that the effects of nanobubble water are not simply dependent on the property of a gas, but are attributed to the characteristic of minute gas bubbles existing as "a gas in a nanobubble state."

### Best Mode for Carrying Out the Invention

### (Preparation for sterilizing or disinfecting tissue)

The preparation for sterilizing or disinfecting a tissue of the present invention is characterized by containing a gas in a nanobubble state.

### <Nanobubbles>

In the present invention, "nanobubble" means gas bubbles having a bubble size (diameter) of nanosize (1 nm or greater and smaller than 1000 nm). In the present invention, the "gas in a nanobubble state" is not particularly limited and can be suitably selected depending on the purpose. Examples thereof include oxygen, ozone, hydrogen, nitrogen, carbon dioxide, natural gases (for example, methane), and so forth. Of these, ozone is preferred as the above-mentioned "gas in a nanobubble state" in view of the tissue disinfecting ability. Furthermore, in the present invention, two or more types of gases can be used as the "gas in a nanobubble state." When two or more types of gases are used, for example, a mixture of nanobubbles consisting of gas A alone and nanobubbles consisting of gas B alone may be used, or nanobubbles containing a mixture of gases A and B may be used. However, gas mixtures are not limited to these examples. For example, a mixture of nanobubbles may be used in which some nanobubbles contain gas A alone, some nanobubbles contain gas B alone, and some nanobubbles contain gases A and B.

The bubble size of nanobubbles can be suitably selected depending on the purpose, but 200 nm or smaller is preferred, and 100 nm or smaller is particularly preferred. In general, the bubble size of 100 nm or smaller is advantageous in view of stability of nanobubbles. It appears that the smaller the bubble size is, the more stable bubbles are in the long-term storage in general.

The bubble size of nanobubbles can be adjusted to a desired size by, for example, utilizing a reverse osmosis membrane or the like. Furthermore, the bubble size of nanobubbles can be assessed by, for example, measurement using a dynamic light scattering photometer or measurement of free radicals. However, when the "gas in a nanobubble state" is ozone, it may damage a reverse osmosis membrane. Therefore, use of the membrane is not necessarily suitable. However, even if the nanobubbles are not passed through a reverse osmosis membrane, it is known that, for example, ozone-nanobubble water in which bubbles having a diameter of 100 nm or smaller account for 95% or more of the gas bubble distribution can be produced.

It is sufficient that at least some of gas bubbles contained in the preparation for sterilizing or disinfecting a tissue of the present invention exist as nanobubbles, and gas bubbles having a larger size (for example, gas bubbles having a bubble size (diameter) of micro size (1 µm or greater and smaller than 1000 µm)) may be contained in addition to the nanobubbles. It is particularly preferred that the concentration of the "gas in a nanobubble state" in preparation for sterilizing or disinfecting a tissue is a saturated concentration. Furthermore, it is particularly preferred that the "gas in a nanobubble state" exists stably in a solution.

When the preparation for sterilizing or disinfecting a tissue is a liquid preparation, the solution constituting the preparation is preferably an aqueous solution, but is not particularly limited and other liquids can be suitably selected depending on the purpose. In the present invention, an aqueous solution containing the "gas in a nanobubble state" is called "nanobubble water." Furthermore, nanobubble water in which the "gas in a nanobubble state" is substantially oxygen alone is suitably called "oxygen-nanobubble water." Nanobubble water in which the "gas in a nanobubble state" is substantially ozone alone is suitably called "ozone-nanobubble water."

Although the nanobubble water of the present invention contains a gas as an internal gas, the bubble size thereof is very minute (for example, smaller than 100 nm). Therefore, the overall amount of the dissolved gas is not particularly increased by containing a certain number of bubbles. In this view, the nanobubble water of the present invention is different from water in which the amount of a dissolved gas is increased. For example, in one embodiment of the present invention, the concentration of dissolved oxygen in oxygen-nanobubble water is maintained at the level similar to that in normal water. Therefore, this oxygen-nanobubble water is different from the prior art which is said to contain several tens or hundreds of times more oxygen (for example, high-concentration oxygen water which may be available with a name of "nanobubble water" and the like). Furthermore, when radicals are measured by the electron spin resonance method using DMPO as a spin-trapping agent, a high peak of hydroxyl radicals can usually be observed in oxygen-nanobubble water, one embodiment of the present invention, if DMPO is added together with hydrochloric acid. On the other hand, in the case of the high-concentration oxygen water, only a weak peak originating in impurities of DMPO can be detected. Thus, nanobubble water can be distinguished from the prior art in view of the dissolved gas concentration and generation of hydroxyl radicals after addition of hydrochloric acid.

### <Other components>

If necessary, the nanobubble water can suitably contain other components in addition to the "gas in a nanobubble state." The other components are not particularly limited and can be suitably selected depending on the purpose, and examples thereof include iron, manganese, salinity, and so forth. Furthermore, salinity concentration, pH, hardness, and the like of the nanobubble water are not particularly limited and can be suitably selected depending on the purpose. These can be adjusted to each desired level, for example, during the production process of nanobubble water described later or after nanobubble water is once produced.

### <Production>

Methods for producing the nanobubble water are not particularly limited and can be suitably selected depending on the purpose. For example, the nanobubble water can be produced according to the methods described in Japanese Patent Laid-Open Nos. 2005-245817, 2005-246294, and 2005-246293 (corresponding to WO2005/084718, WO2005/084786, and WO2005/085141, respectively), and the like. The production methods described in the above-mentioned publications are preferred because nanobubble water in which a "gas in a nanobubble state" exists stably over a long period of several months or longer and is not eliminated from an aqueous solution can be produced.

Furthermore, during the production process of the nanobubble water, it is preferable to add iron, manganese, salinity, and the like to an aqueous solution to be used.

In the production process of the nanobubble water, the salinity concentration in an aqueous solution to be used is preferably 0.2 to 3.0% by mass, more preferably 0.8 to 1.2% by mass. In general, when the salinity concentration is in the range of 0.8 to 1.2% by mass, a gas nucleus of a nanobubble can be easily generated, resulting in an excellent production efficiency of nanobubble water. The above-mentioned salinity concentration can be measured by, for example, using known salinity concentration measuring instruments.

In the production process of the nanobubble water, it is generally thought that pH, hardness, and the like of an aqueous solution to be used do not affect the production efficiency of nanobubbles as greatly as the salinity concentration, but usually pH is preferably 7 to 8, and hardness is preferably 20 to 30. The above-mentioned pH, hardness, and the like can be measured by, for example, using known pH meters, known hardness testing machines, and the like.

More specifically, the nanobubble water can be produced by, for example, producing microbubbles of 50 µm or smaller using hard water (groundwater) having a salinity concentration of 1.0% by mass as a raw material and then rapidly crushing the bubbles by applying pressure. By further passing this nanobubble water twice through a 10-Å reverse osmosis membrane, nanobubble water having a salinity concentration of 0% by mass can be produced (oxygen-nanobubble water having a salinity concentration of 0% by mass is known as "Naga no Shizuku" [NAGA Co., Ltd.] approved by the Ministry of Health, Labour, and Welfare as drinking water). Meanwhile, nanobubble water before being passed through a 10-Å reverse osmosis membrane can be used as nanobubble water having a salinity concentration of 1.0% by mass. Nanobubble water having a salinity concentration of 0 to 1.0% by mass can be provided by changing the mixing ratio of these two nanobubble waters. When ozone-nanobubble water is passed through a reverse osmosis membrane, the device may be melted and broken, and use of a reverse osmosis membrane is not preferable.

Nanobubble water obtained as described above may be used, for example, as the preparation for sterilizing or disinfecting a tissue as it is, or as the preparation for sterilizing or disinfecting a tissue by using other components in combination. For example, it is expected that the tissue sterilizing or disinfecting ability can be further improved by adding a preparation for sterilizing or disinfecting a tissue having a conventional composition to the above-mentioned nanobubble water or using the above-mentioned nanobubble water to produce a preparation for sterilizing or disinfecting a tissue having a conventional composition. Furthermore, the nanobubble water can be used in combination with a conventional drug which can be used for sterilization or disinfection of tissues or the like. Accordingly, such preparations for sterilizing or disinfecting a tissue using the nanobubble water as a part thereof are also encompassed in the scope of the preparation for sterilizing or disinfecting a tissue of the present invention.

### <Tissues>

Target "tissues" for which the preparation for sterilizing or disinfecting a tissue of the present invention is used are not particularly limited and can be suitably selected depending on the purpose. Examples thereof include epithelial tissues, connective tissues, muscle tissues, nerve tissues, and so forth. Furthermore, in the present specification, the "tissue" is a concept that also includes "cells" constituting the tissue and an "organ" constituted by the tissue. Examples of the "cells" include epidermal cells, pancreatic parenchymal cells, pancreatic duct cells, hepatocytes, blood cells, cardiac muscle cells, skeletal muscle cells, osteoblasts, skeletal myoblasts, nerve cells, vascular endothelial cells, pigment cells, smooth muscle cells, fat cells, bone cells, cartilage cells, and so forth. Furthermore, examples of the above-mentioned "organ" include skin, blood vessels, cornea, kidneys, heart, liver, umbilical cord, intestines, nerves, lungs, placenta, pancreas, brain, distal portions of the extremities, retina, and so forth.

The tissue may be derived from any organism, and the organism can be suitably selected depending on the purpose. Above all, the tissue can be derived from mammals, particularly from humans.

The tissue may exist in the body or outside the body (for example, a cultured tissue, etc.).

### <Sterilization and disinfection>

In the present invention, both "sterilization" and "disinfection" of a tissue mean to kill or reduce a pathogenic or harmful microorganism such as a bacterium or a virus, or suppress activities of the microorganism in the tissue. Furthermore, in the present specification, the "sterilization" and "disinfection" are concepts also including, for example, killing and removal of all microbes, removal of microbes, microbial reduction, antibacterial treatment, microbiostasis, prevention of molds, and so forth.

### <Applications>

Methods of using the preparation for sterilizing or disinfecting a tissue of the present invention are not particularly limited and can be suitably selected depending on the purpose. For example, the preparation can be used by bringing the preparation into contact with the tissue infected with a pathogenic or harmful microorganism such as a bacterium or virus by an arbitrary method. In the tissue sterilizing or disinfecting method of the present invention, methods of applying a "gas in a nanobubble state" are not particularly limited and can be suitably selected depending on the purpose. For example, the gas can be applied by bringing an effective amount of nanobubble water into contact with the above-mentioned tissue infected with a pathogenic or harmful microorganism such as a bacterium or a virus by an arbitrary method.

Methods for storing the preparation for sterilizing or disinfecting a tissue are not particularly limited and can be suitably selected depending on the purpose. When ozone is used as the "gas in a nanobubble state," it is recommended to avoid an ultraviolet ray and store the preparation in the dark refrigerator to prevent deterioration.

Since the preparation for sterilizing or disinfecting a tissue has an excellent tissue sterilizing or disinfecting ability, it can be suitably used for, for example, therapeutic or prophylactic treatment of various diseases and conditions caused by infection with a pathogenic or harmful microorganism such as a bacterium or a virus (for example, therapeutic or prophylactic treatment of periodontal diseases or the like). In the method for therapeutic or prophylactic treatment of periodontal disease of the present invention, methods of applying a "gas in a nanobubble state" are not particularly limited and can be suitably selected depending on the purpose. For example, the gas can be applied by bringing an effective amount of nanobubble water into contact with an affected area by an arbitrary method. The above-mentioned periodontal diseases are not particularly limited, and examples thereof include gingivitis, periodontitis, and so forth.

### Example 1

Hereafter, examples of the present invention will be explained. However, the scope of the present invention is not limited to these examples.

### (Example 1: Evaluation of tissue sterilizing or disinfecting effects of ozone-nanobubble water)

Ozone-nanobubble water, one embodiment of the preparation for sterilizing or disinfecting a tissue of the present invention, was prepared with reference to the production method described in Japanese Patent Laid-Open No. 2005-246293 (corresponding to WO2005/085141). Specifically, hard water (groundwater) having a salinity concentration of 1.0% by mass was used as a raw material and microbubbles of 50 µm or smaller were prepared and then rapidly crushed by applying pressure to prepare the ozone-nanobubble water. The tissue sterilizing or disinfecting effects of the obtained ozone-nanobubble water were clinically examined in patients suffering from periodontal diseases (gingivitis and periodontitis) as subjects.

### <Method>

The subjects included four patients suffering from periodontal diseases (gingivitis and periodontitis). These patients were allowed to rinse out their mouth with 20 mL of the above-mentioned ozone-nanobubble water for 20 seconds each time twice daily, in the morning and evening, for two weeks. Changes in clinical symptoms (depth of the periodontal pocket and hemorrhage upon probing -- i.e., measurement of depth of the periodontal pocket) were observed. In this example, to purely evaluate the effects of the above-mentioned ozone-nanobubble water, brushing was not instructed deliberately, and whether clinical symptoms would be improved by rinsing with the ozone-nanobubble water alone was examined.

### <Results>

The results are shown in Tables 1 and 2. Table 1 shows changes over time in the proportion (%) of sites of hemorrhage upon probing. Table 2 shows the percentage of periodontal pockets where the depths were reduced by 1 mm or more among the pockets having an original depth of 3 mm or greater, and the number of these pockets.

**[Table 1]**

| | Whole jaw | | | Upper jaw | | | Lower jaw | | |
|---|---|---|---|---|---|---|---|---|---|
| Subject | 0 | I | II | 0 | I | II | 0 | I | II |
| 1 | 45.8 | 12.5 | 8.9 | 47.6 | 15.5 | 8.3 | 44 | 9.5 | 9.5 |
| 2 | 37.1 | 7.5 | 5.4 | 38.9 | 8.9 | 3.3 | 35.4 | 6.3 | 7.3 |
| 3 | 18.8 | 4.7 | 4.7 | 25 | 7.3 | 7.3 | 12.5 | 2.1 | 2.1 |
| 4 | 35.8 | 16 | 18 | 25.6 | 14.1 | 9 | 45.2 | 17.9 | 9 |
| Mean | 34.375 | 10.175 | 9.25 | 34.275 | 11.45 | 6.975 | 34.275 | 8. 95 | 6.975 |

| | | | | | | | | | |
|---|---|---|---|---|---|---|---|---|---|
| Proportion (%) of sites of hemorrhage upon probing | | | | | | | | | |

**[Table 2]**

| Subject | Percentage of periodontal pockets with reduction of the depth by >1 mm (%) | No. of the pockets with reduction of the depth by >1 mm / No. of pockets having an original depth of >3 mm |
|---|---|---|
| 1 | 62.3 | 38/61 |
| 2 | 82 | 50/61 |
| 3 | 52.4 | 22/42 |
| 4 | 83 | 44/53 |
| Mean | 69.925 | - |

The results in Table 1 demonstrate that the proportions of hemorrhage sites were clearly reduced, showing that the mean proportion of hemorrhage sites in the upper and lower jaws (whole jaws) changed from approx. 34.4% on the rinse start day (0) to approx. 10.2 and 9.3% at one week (I) and two weeks (II), respectively.
Further, the results in Table 2 demonstrate that the depth of the periodontal pocket was clearly improved, showing that the mean proportion of periodontal pockets with reduction of the depth by 1 mm or more among the periodontal pockets having an original depth of 3 mm or greater (percentage of reductions by 1 mm or more) was approx. 70.0%.

The above results demonstrated that clinical symptoms of periodontal diseases (gingivitis and periodontitis) were dramatically improved by rinsing out the mouth with nanobubble water. This suggests that periodontal disease-causing bacteria existing in plaques were eliminated by sterilization or disinfection, and periodontal tissues were regenerated by the action of nanobubble water. Therefore, these results suggested that nanobubble water had excellent tissue sterilizing or disinfecting effects.

Furthermore, the above-mentioned results are very surprising taking into account that the plaque control records (percentage of dental plaques attached in a quarter of one tooth) of the patients were 80% or higher at the initial visit (before the start of rinsing), and brushing after the visit was not instructed. Generally in treatment of periodontal diseases, the plaque control record needs to be reduced to 20% or lower first. In this experiment, clinical symptoms were dramatically improved by rinsing with nanobubble water alone without instructing brushing. This result suggests that nanobubble water is very promising as an agent effective for therapeutic or prophylactic treatment of periodontal diseases and can be widely applied for therapeutic or prophylactic treatment of various diseases caused by infection with microorganisms by utilizing the excellent tissue sterilizing or disinfecting ability thereof.

### Industrial Applicability

The preparation for sterilizing or disinfecting a tissue of the present invention can be suitably used for therapeutic or prophylactic treatment of various diseases caused by infection with microorganisms (for example, periodontal diseases).

## Claims

1. A preparation for sterilizing or disinfecting a tissue, **characterized by** comprising a gas in a nanobubble state.

2. The preparation for sterilizing or disinfecting a tissue according to claim 1, **characterized in that** the gas in a nanobubble state is ozone.

3. A liquid preparation for sterilizing or disinfecting a tissue, **characterized by** comprising ozone-nanobubble water.

4. An agent for therapeutic or prophylactic treatment of a periodontal disease, **characterized by** comprising a gas in a nanobubble state.

5. The agent for therapeutic or prophylactic treatment of a periodontal disease according to claim 4, **characterized in that** the gas in a nanobubble state is ozone.

6. An agent for therapeutic or prophylactic treatment of a periodontal disease, **characterized by** comprising ozone-nanobubble water.

7. A method for sterilizing or disinfecting a tissue, **characterized by** applying an effective amount of a gas in a nanobubble state.

8. The method for sterilizing or disinfecting a tissue according to claim 7, **characterized in that** the gas in a nanobubble state is ozone.

9. A method for sterilizing or disinfecting a tissue, **characterized by** applying an effective amount of ozone-nanobubble water.

10. A method for therapeutic or prophylactic treatment of a periodontal disease, **characterized by** applying an effective amount of a gas in a nanobubble state.

11. The method for therapeutic or prophylactic treatment of a periodontal disease according to claim 10, **characterized in that** the gas in a nanobubble state is ozone.

12. A method for therapeutic or prophylactic treatment of a periodontal disease, **characterized by** applying an effective amount of ozone-nanobubble water.

13. Use of a gas in a nanobubble state for producing a preparation for sterilizing or disinfecting a tissue.

14. The use according to claim 13, wherein the gas in a nanobubble state is ozone.

15. Use of a gas in a nanobubble state for producing an agent for therapeutic or prophylactic treatment of a periodontal disease.
